# EUROPEAN PATENT APPLICATION

(11) **EP 4 844 768 A1**
(43) Date of publication of application: **25.11.2026**
(21) Application number: 25199158.4
(22) Date of filing: 29.08.2025
(51) Int. Cl.: G16B 5/30, G16B 15/30, G16B 40/20, G16C 10/00, G16C 20/50

(54) **COMPUTER-IMPLEMENTED METHOD FOR FACILITATING A MOLECULAR DYNAMICS SIMULATION FOR DRUG DISCOVERY**

(30) Priority: 20.05.2025 TW 114118846
(71) Applicant: Horuseye Technology Co., Ltd., New Taipei City 23144 (TW)
(72) Inventor: HSIAO, Yung-Chin, 23144 New Taipei City (TW); LIU, Chih-Fan, 23144 New Taipei City (TW); CHANG, Ching-Ya, 23144 New Taipei City (TW)
(74) Representative: Michalski Hüttermann & Partner mbB

(57) **Abstract**

A computer-implemented method for facilitating a molecular dynamics simulation for drug discovery is provided. The computer-implemented method comprises: performing a molecular dynamics simulation process with respect to a target molecule and a test molecule until a dissociation condition is satisfied; and adaptively performing a stochastic perturbation process during the performing of the molecular dynamics simulation process. The stochastic perturbation process comprises: obtaining states of the target molecule and the test molecule in the molecular dynamics simulation process; determining a perturbation set; and obtaining perturbed states of the target molecule and the test molecule according to the perturbation set, wherein the molecular dynamics simulation process proceeds according to the perturbed states of the target molecule and the test molecule. In response to that the dissociation condition is not satisfied, the stochastic perturbation process is performed at a perturbation time interval to expedite the molecular dynamics simulation process.

## Description

### BACKGROUND OF THE INVENTION

### 1. Field of the Invention

The present disclosure relates to a computer-implemented method for molecular dynamics simulation, and particularly relates to a computer-implemented method for facilitating molecular dynamics simulation for drug discovery.

### 2. Description of the Related Art

The residence time of binding between a test molecule and a target molecule plays a crucial role in evaluating its efficacy. However, simulating a dissociation process to determine the residence time using molecular dynamics simulation is computationally challenging.

In the ever-developing field of computer-aided drug design, the molecular druggability is critical. Various methods have been established to analyze test molecules and their interactions with target receptors. Key metrics employed in these studies include estimation of inhibitory concentration (IC50), equilibrium dissociation constant (K_{d}), and inhibitory constant (Kᵢ). Over the past decade, the understanding of drug efficacy and pharmacological activity has advanced significantly. It is now widely recognized that the principal determinant of drug efficacy is not solely its binding affinity to the target receptor. Instead, the kinetics of drug-receptor interactions plays a crucial role. Specifically, the lifetime or residence time of a drug on an intended target receptor (defined as a reciprocal of the dissociation constant (*k*_{off}) derived from binding kinetics) has shown a greater correlation with drug efficacy.

The binding kinetics is usually determined by experimental studies. However, computer simulation methods are rapidly improving the estimation of the binding kinetics. Spontaneous ligand binding and dissociation are slow processes, making conventional molecular dynamics simulations computationally expensive. While specialized hardware such as Anton supercomputer has advanced molecular dynamics (MD) simulation capabilities, it is still not available to most users. In addition, theoretical methods combined with graphics processing unit (GPU) supercomputing has developed enhanced sampling methods to capture critical binding and disassociation events. These include Markov state models, guided molecular dynamics, weighted ensemble methods, random acceleration molecular dynamics, and Ligand Gaussian accelerated molecular dynamics (LiGaMD). Among these innovative methods, a τ-RAMD method based on random acceleration molecular dynamics (RAMD) simulation is proposed, which can be used for rapidly estimating relative residence time.

### BRIEF SUMMARY OF THE INVENTION

One objective of the present disclosure is to provide a computer-implemented technology for facilitating a molecular dynamics simulation for drug discovery. According to the technology, in the performing of a molecular dynamics simulation process, a stochastic perturbation process is adaptively performed to expedite the performing of the molecular dynamics simulation process.

In order to achieve at least the above objective, the present disclosure provides embodiments of a computer-implemented method for facilitating a molecular dynamics simulation for drug discovery. The computer-implemented method includes: performing a molecular dynamics simulation process with respect to a target molecule and a test molecule until a dissociation condition is satisfied; and adaptively performing a stochastic perturbation process during the performing of the molecular dynamics simulation process. The stochastic perturbation process includes: obtaining states of the target molecule and the test molecule in the molecular dynamics simulation process before performing the stochastic perturbation process; determining a perturbation set based on the states of the target molecule and the test molecule; and performing perturbation on the target molecule and the test molecule according to the perturbation set to obtain perturbed states of the target molecule and the test molecule, wherein the molecular dynamics simulation process proceeds according to the perturbed states of the target molecule and the test molecule. In response to that the dissociation condition is not satisfied, the stochastic perturbation process is performed at a perturbation time interval in the performing of the molecular dynamics simulation process, so as to expedite the molecular dynamics simulation process.

In an embodiment of the computer-implemented method, the perturbation set includes a group of acceleration stochastic perturbations for each atom of the test molecule.

In an embodiment of the computer-implemented method, the perturbation set includes a group of velocity stochastic perturbations for each atom of the test molecule.

In an embodiment of the computer-implemented method, the perturbation set includes a group of displacement stochastic perturbations for each atom of the test molecule.

In an embodiment of the computer-implemented method, the perturbation time interval of the stochastic perturbation process is a fixed time interval.

In an embodiment of the computer-implemented method, the perturbation set includes a group of stochastic perturbations, and each stochastic perturbation in the group of stochastic perturbations has a same perturbation magnitude.

In an embodiment of the computer-implemented method, each stochastic perturbation in the group of stochastic perturbations is distributed within a cone angle with respect to the test molecule based on a guiding vector.

In an embodiment of the computer-implemented method, the perturbation set includes a group of stochastic perturbations that are distributed within a cone angle of the test molecule and compatible with a pocket of the target molecule.

In an embodiment of the computer-implemented method, the perturbation set includes a group of stochastic perturbations that are stochastically generated based on the guiding vector and adaptively distributed along a pathway departing from a pocket of the target molecule.

In an embodiment of the computer-implemented method, the guiding vector is a configurable vector.

In an embodiment of the computer-implemented method, when the molecular dynamics simulation process starts, the guiding vector is configured in response to an input indication of a user through an application.

In an embodiment of the computer-implemented method, the application program is an application program for one of augmented reality (AR), virtual reality (VR), mixed reality (MR) or extended reality (XR) technologies, and configures the guiding vector in response to an input indication from a hand interaction action of the user.

In an embodiment of the computer-implemented method, the guiding vector is an adaptive guiding vector, and the adaptive guiding vector is updated to a first guiding vector when the test molecule enters a region with respect to a starting point of the first guiding vector.

In an embodiment of the computer-implemented method, after the adaptive guiding vector is updated to the first guiding vector, the adaptive guiding vector is further updated to a second guiding vector when the test molecule enters a region with respect to a starting point of the second guiding vector.

In an embodiment of the computer-implemented method, the guiding vector is a vector from a center of geometry of the target molecule to a center of geometry of the test molecule.

In an embodiment of the computer-implemented method, the guiding vector is a vector from a center of mass of the target molecule to a center of mass of the test molecule.

In an embodiment of the computer-implemented method, the dissociation condition is whether an inequality Dₜ > Dₛₜₒₚ is satisfied, wherein Dₛₜₒₚ = R₁ + R₂ + D_{buffer}, Dₜ represents a distance from a center point of the target molecule to a center point of the test molecule at a time point in the performing of the molecular dynamics simulation process; R₁ represents the maximum distance from the center point of the target molecule to an outermost atom of the target molecule when the molecular dynamics simulation process starts; R₂ represents a maximum distance from the center point of the test molecule to an outermost atom of the test molecule when the molecular dynamics simulation process starts; and D_{buffer} represents a buffer distance.

In an embodiment of the computer-implemented method, the buffer distance is 4-10Å (note: 1Å is 10⁻¹⁰ m).

In order to achieve at least the objective above, the present disclosure further provides a computer program product which includes a plurality of instructions, and the plurality of instructions are used for causing at least one computing device to execute any one of the embodiments of the computer-implemented method for facilitating the molecular dynamics simulation for drug discovery or a combination thereof. In an embodiment, the computer program product includes a non-transient computer storage medium which stores the plurality of instructions.

In order to achieve at least the objective above, the present disclosure further provides a computing system, including: at least one computing device; and at least one storage device which stores a plurality of instructions, wherein when executed by the at least one computing device, the instructions cause the at least one computing device to execute any one of the embodiments of the computer-implemented method for facilitating the molecular dynamics simulation for drug discovery or a combination thereof.

### BRIEF DESCRIPTION OF THE DRAWINGS

FIG. 1 is a schematic sequence diagram illustrating an embodiment of a computer-implemented method for facilitating a molecular dynamics simulation for drug discovery.
FIG. 2 is a schematic flowchart illustrating an embodiment of a molecular dynamics simulation process.
FIG. 3 is a schematic flowchart illustrating an embodiment of a stochastic perturbation process.
FIG. 4 is a schematic block diagram illustrating some embodiments of a computing system for implementing molecular dynamics simulation based on FIG. 1.
FIG. 5 is a schematic flowchart illustrating an embodiment of a method for applying adaptively stochastic perturbations in molecular dynamics simulation (ASPMDS) to atoms of a test molecule.
FIG. 6 is a schematic flowchart illustrating another embodiment of a method for applying adaptively stochastic perturbations in molecular dynamics simulation (ASPMDS) to atoms of a test molecule.
FIG. 7 is a schematic diagram illustrating an embodiment of a perturbation protocol.
FIG. 8 is a schematic diagram illustrating an embodiment of an atom being subject to acceleration perturbation.
FIG. 9 is a schematic diagram illustrating an embodiment of an atom being subject to velocity perturbation.
FIG. 10 is a schematic diagram illustrating an embodiment of an atom being subject to displacement perturbation.
FIG. 11 is a schematic diagram illustrating an embodiment of adaptively updating a guiding vector.
FIGS. 12A, 12B, and 12C are schematic diagrams illustrating embodiments of a group of stochastic unit vectors distributed about a reference vector (0,0,1) within respective specified angles Psi_max.
FIG. 13 is a schematic flowchart illustrating a group of uniformly-distributed stochastic unit vectors generated in a special coordinate system.

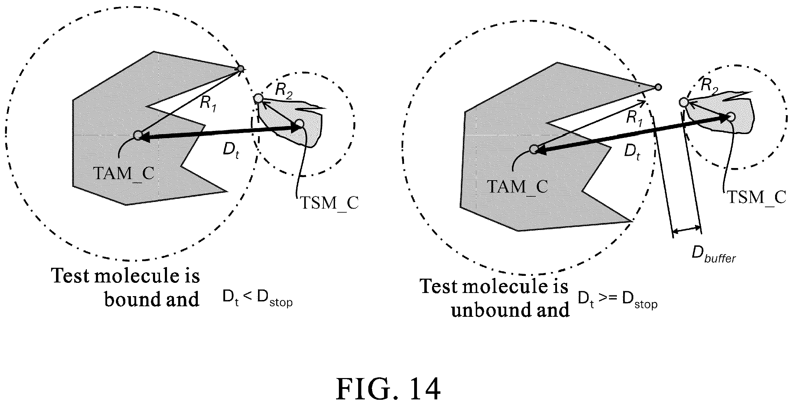

FIG. 14 is a schematic diagram illustrating an embodiment of a stop condition.
FIG. 15 is a schematic flowchart illustrating operations of an adaptively stochastic perturbations in molecular dynamics simulation (ASPMDS).
FIG. 16 is a schematic flowchart illustrating adaptively updating a guiding vector.
FIG. 17 is a diagram illustrating regression models of τ-RAMD, ASPMDS-180, and ASPMDS 90.

### DETAILED DESCRIPTION OF THE INVENTION

In order to fully understand the objectives, features and efficacy of the present disclosure, the present disclosure is described in detail by specific embodiments and in cooperation with the accompanying drawings, as follows:

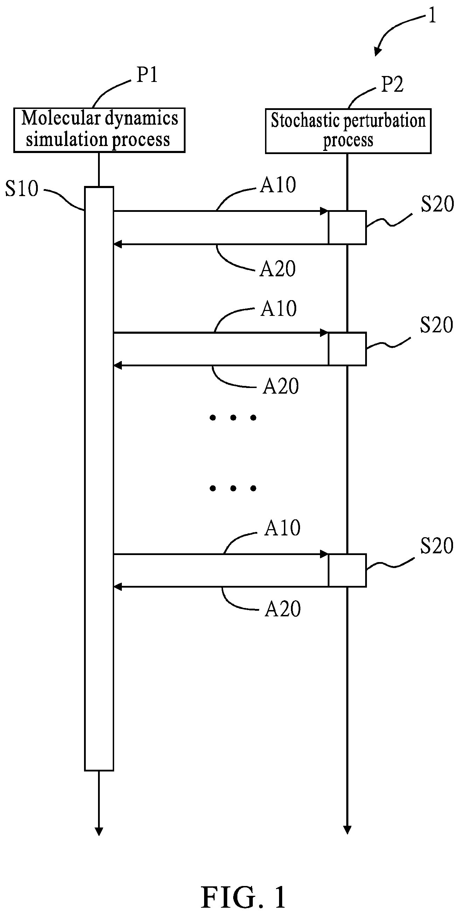

FIG. 1 is a schematic sequence diagram illustrating an embodiment of a computer-implemented method for facilitating a molecular dynamics simulation for drug discovery. As shown in FIG. 1, the computer-implemented method for facilitating the molecular dynamics simulation for drug discovery (or method 1 for short) relates to a molecular dynamics simulation process P1 and a stochastic perturbation process P2 executed in a computing system. A downward arrow in FIG. 1 represents a time axis. The molecular dynamics simulation process P1 is to perform molecular dynamics simulation on a target molecule and a test molecule. The target molecule is, for example, a receptor, protein, or deoxyribonucleic acid (DNA). A molecule whose dissociation process with respect to the target molecule is to be tested through the molecular dynamics simulation process P1 is referred to as the test molecule; and the test molecule may be, for example, a ligand, a drug molecule, a drug-like molecule, protein or a protein drug. The implementation of the present disclosure is not limited to the above examples of the target molecule and the test molecule. As shown in FIG. 1, the method 1 includes operations S10 and S20.

Operation S10 includes: performing the molecular dynamics simulation process P1 with respect to the target molecule and the test molecule until the dissociation condition of the molecular dynamics simulation process P1 is satisfied.

Operation S20 includes: adaptively performing the stochastic perturbation process P2 in the performing of the molecular dynamics simulation process P1 (as shown in operation S10).

In the method 1, the molecular dynamics simulation process P1 proceeds according to perturbed states of the target molecule and the test molecule. In response to the case that the dissociation condition is not satisfied, the stochastic perturbation process P2 is executed at a perturbation time interval in the performing of the molecular dynamics simulation process P1, so as to expedite the molecular dynamics simulation process P1.

In the method 1 in FIG. 1, for example, the molecular dynamics simulation process P1 is implemented based on a computer simulation method for analyzing physical motion of a large number of atoms and molecules by the Newton' S law, and the interactions between the target molecule (such as a receptor, which is usually a large protein molecule) and the test molecule (such as a ligand that can bind to the receptor, which is usually a small molecule) are simulated on an atomic level. It is to be noted that in the present disclosure, the implementation of the molecular dynamics simulation process P1 is not limited, and as long as appropriate, any algorithm that can implement the molecular dynamics simulation of the target molecule and the test molecule can be used for implementing the molecular dynamics simulation process P1.

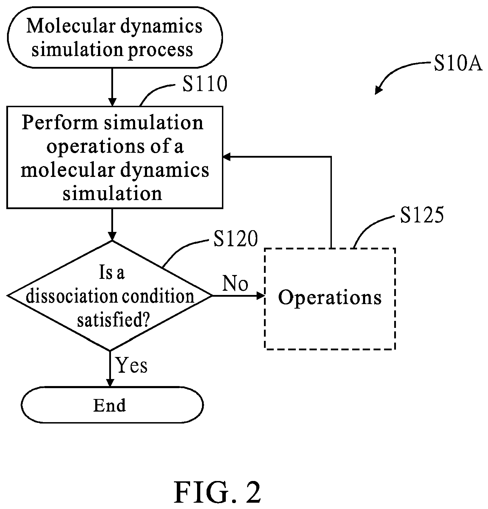

With respect to the stop of operation S10, in an embodiment of the molecular dynamics simulation process shown in FIG. 2, when the dissociation condition of the molecular dynamics simulation process S10A is satisfied, such as the determination result in operation S120 being yes, the molecular dynamics simulation process S10A is stopped; and when the dissociation condition of the molecular dynamics simulation process S10A is not satisfied, such as the determination result in operation S120 being no, the molecular dynamics simulation process S10A proceeds simulation operations, as shown in operation S110.

In some embodiments of the method 1 based on FIG. 1, the dissociation condition is whether an inequality Dₜ > Dₛₜₒₚ is satisfied, where Dₛₜₒₚ = R₁ + R₂ + D_{buffer}, Dₜ represents a distance from the center point of the target molecule to the center point of the test molecule at a time point in the performing of the molecular dynamics simulation process; R₁ represents the maximum distance from the center point of the target molecule to the outermost atom of the target molecule when the molecular dynamics simulation process starts; R₂ represents the maximum distance from the center point of the test molecule to the outermost atom of the test molecule when the molecular dynamics simulation process starts; and D_{buffer} represents a buffer distance. In some examples, the buffer distance is 4-10Å.

With respect to operation S20, the stochastic perturbation process P2 can obtain the states (shown as an arrow A10) of the target molecule and the test molecule in the molecular dynamics simulation process P1, and provides or outputs the perturbed states (shown as an arrow A20) of the target molecule and the test molecule to the molecular dynamics simulation process P1. The molecular dynamics simulation process P1 proceeds according to the perturbed states of the target molecule and the test molecule. As shown in FIG. 1, the stochastic perturbation process P2 is performed in the molecular dynamics simulation process P1 for multiple times, thereby expediting the molecular dynamics simulation process P1, as well as decreasing the performing time.

In addition, in operation S20, the stochastic perturbation process P2 can be adaptively performed in the molecular dynamics simulation process P1, that is, the timing or manner of performing the stochastic perturbation process P2 in the molecular dynamics simulation process P1 may vary depending on different conditions: for example, the stochastic perturbation process P2 is actively performed; or the stochastic perturbation process P2 is performed in response to an external request; or the stochastic perturbation process P2 is performed after a certain condition is satisfied, or the stochastic perturbation process P2 is performed in other appropriate manners.

A plurality of embodiments are further provided below to illustrate the implementation of the stochastic perturbation process P2.

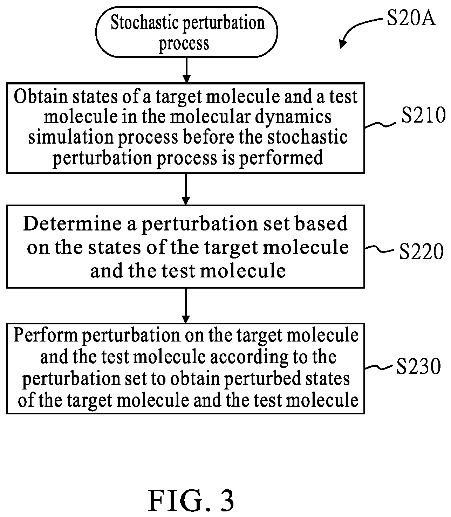

FIG. 3 is a schematic flowchart illustrating an embodiment of a stochastic perturbation process. As shown in FIG. 3, the stochastic perturbation process S20A is an embodiment of the stochastic perturbation process P2 in FIG. 1. The stochastic perturbation process S20A includes operations S210 to S230.

Operation S210 includes: obtaining the states of the target molecule and the test molecule in the molecular dynamics simulation process (such as P1) before the stochastic perturbation process S20A is performed.

Operation S220 includes: determining a perturbation set based on the states of the target molecule and the test molecule.

Operation S230 includes: performing a perturbation operation on the target molecule and the test molecule according to the perturbation set to obtain the perturbed states of the target molecule and the test molecule.

Through the embodiment shown in FIG. 3, the molecular dynamics simulation process P1 proceeds the molecular dynamics simulation after the perturbed states of the target molecule and the test molecule are obtained, thereby facilitating the molecular dynamics simulation process.

In some embodiments of the method 1 based on FIG. 1 and FIG. 3, the perturbation time interval of the stochastic perturbation process P2 is a fixed time interval. For example, as shown in FIG. 1, under the condition that the dissociation condition is not satisfied, after operation S20 is performed for the first time, operation S20 is performed again at each perturbation time interval.

In addition, with respect to the molecular dynamics simulation process P1, for example, in FIG. 2, after the determination result in operation S120 is no, for example, in the process of operation S125 (for example, preparation for next simulation), the stochastic perturbation process P2 is performed; or in the process of simulation operations S110 of performing the molecular dynamics simulation. For example, the molecular dynamics simulation process P1 and the stochastic perturbation process P2 may be implemented as independent process or thread; or the stochastic perturbation process P2 may be implemented as sub-processing, thread, sub-program or function of the molecular dynamics simulation process P1. The implementation of the present disclosure is not limited by the manner of performing or implementation of the molecular dynamics simulation simulation process P1 and stochastic perturbation process P2.

Further, in operation S220, the "perturbation set" serves as a generic term or collective noun of a plurality of "stochastic perturbations" to represent stochastic perturbations of a physical quantity with respect to motion associated with each atom of the test molecule at a time (such as the current perturbation). One of acceleration, velocity or displacement may be taken to define the physical quantity in implementation. Therefore, a stochastic perturbation represents a vector associated with the physical quantity, which represents the direction and magnitude of a perturbation. In implementation, the "perturbation set" may be generated, for example, in accordance with settings of a perturbation magnitude, a perturbation time interval and/or a perturbation direction. A plurality of embodiments are provided below to describe the implementations of determining the perturbation set of operation S220 in FIG. 3.

In some embodiments of the method 1 based on FIG. 1 and FIG. 3, the perturbation set includes a group of acceleration stochastic perturbations for each atom of the test molecule.

In some embodiments of the method 1 based on FIG. 1 and FIG. 3, the perturbation set includes a group of velocity stochastic perturbations for each atom of the test molecule.

In some embodiments of the method 1 based on FIG. 1 and FIG. 3, the perturbation set includes a group of displacement stochastic perturbations for each atom of the test molecule.

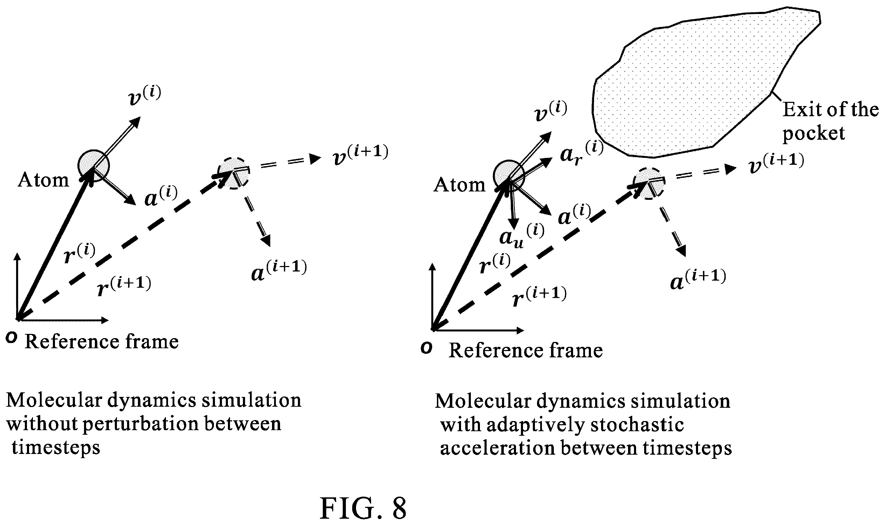

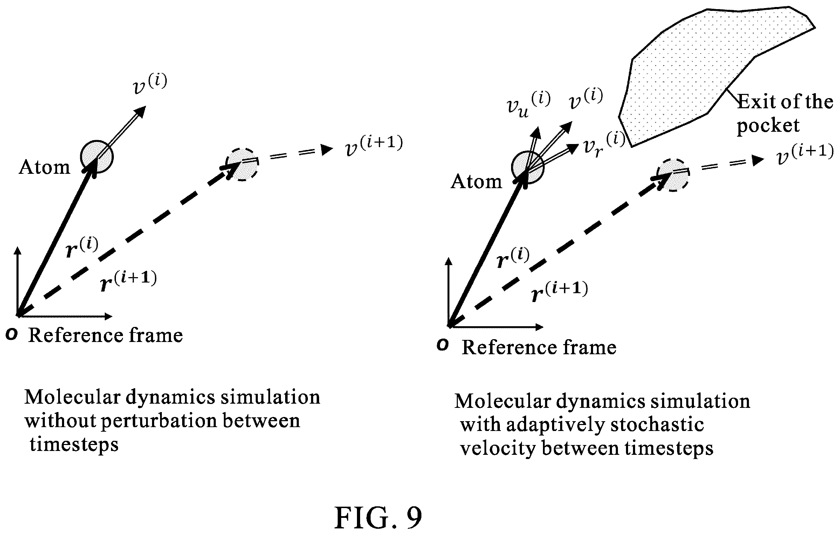

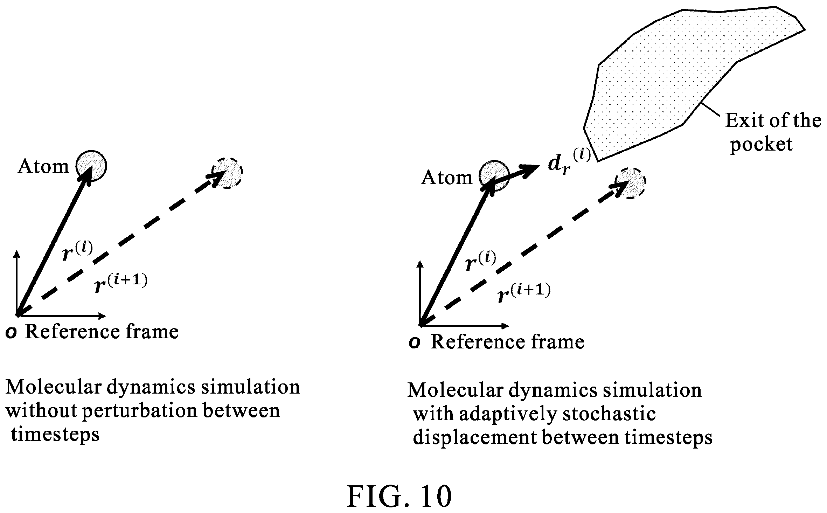

In some embodiments of the method 1 based on FIG. 1 and FIG. 3, the perturbation set includes a group of stochastic perturbations, and each stochastic perturbation in the group of stochastic perturbations has the same perturbation magnitude. For example, the group of stochastic perturbations are one kind of acceleration stochastic perturbations (shown in FIG. 8 as aᵣ), velocity stochastic perturbations (shown in FIG. 9 as vᵣ), or displacement stochastic perturbations (shown in FIG. 10 as dᵣ) for each atom of the test molecule.

In some embodiments of the method 1 based on FIG. 1 and FIG. 3, each stochastic perturbation in the group of stochastic perturbations is distributed within a cone angle with respect to the test molecule based on a guiding vector.

In some embodiments of the method 1 based on FIG. 1 and FIG. 3, the perturbation set includes a group of stochastic perturbations (such as the above-mentioned acceleration stochastic perturbation, velocity stochastic perturbation, or displacement stochastic perturbation) that are distributed within a cone angle of the test molecule and compatible with a pocket of the target molecule.

In some embodiments of the method 1 based on FIG. 1 and FIG. 3, the perturbation set includes a group of stochastic perturbations (such as the above-mentioned acceleration stochastic perturbation, velocity stochastic perturbation, or displacement stochastic perturbation) that are stochastically generated based on the guiding vector and adaptively distributed along a pathway departing from a pocket of the target molecule.

In some embodiments of the method 1 based on FIG. 1 and FIG. 3, the guiding vector is a configurable vector. For example, the guiding vector is configured in response to input indications of a user through an application program of the computing system.

In some embodiments of the method 1 based on FIG. 1 and FIG. 3, the guiding vector is an adaptive guiding vector, and the adaptive guiding vector is updated to a first guiding vector when the test molecule enters a region with respect to a starting point of the first guiding vector.

In some embodiments of the method 1 based on FIG. 1 and FIG. 3, after the adaptive guiding vector is updated to the first guiding vector, the adaptive guiding vector is further updated to a second guiding vector when the test molecule enters a region with respect to a starting point of the second guiding vector.

In some embodiments of the method 1 based on FIG. 1 and FIG. 3, the guiding vector is a vector from the center of geometry of the target molecule to the center of geometry of the test molecule.

In some embodiments of the method 1 based on FIG. 1 and FIG. 3, the guiding vector is a vector from the center of mass of the target molecule to the center of mass of the test molecule.

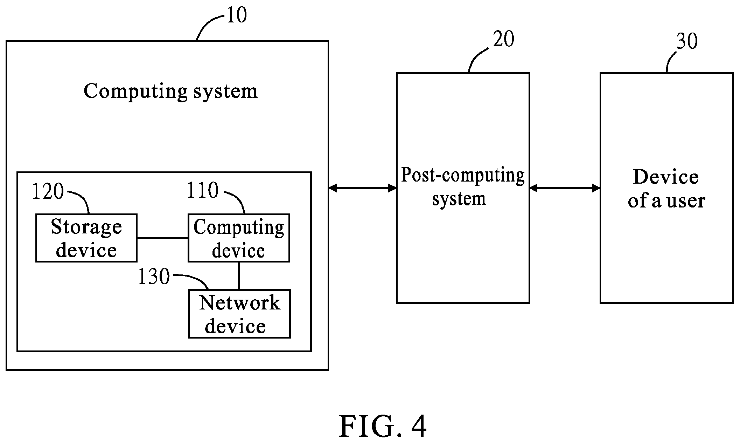

FIG. 4 is a schematic block diagram illustrating some embodiments of a computing system for implementing molecular dynamics simulation based on FIG. 1. In an embodiment, the computing system 10 includes at least one computing device 110; and at least one storage device 120 that stores a plurality of instructions; and when executed by the at least one computing device 110, the instructions cause the at least one computing device 110 to perform any one of the embodiments of the computer-implemented method for facilitating the molecular dynamics simulation for drug discovery or a combination thereof.

As shown in FIG. 4, in another embodiment, the computing system 10 may further include a network device 130. The network device 130 is electrically coupled to the computing device 110 for network communication. The network device 130 may be a wireless or wired communication circuit, which is, for example, used for establishing communication with at least one of a broadband network, an optical fiber network, a wireless area network and a mobile communication network so as to realize communication with the network. The computing device 110 includes at least one of a microprocessor, a graphics processing unit and any circuit with computing function. In addition, the storage device 120 is, for example, a memory device, a hard disk, a solid-state drive or a cloud storage space or a combination thereof, and can be used for storing instruction code and data of the molecular dynamics simulation process P1 and the stochastic perturbation process P2. For example, the computing system 10 may be implemented through one computer or a plurality of computers.

As shown in FIG. 4, in yet another embodiment, the computing system 10 may further communicate with a post-computing system 20 (such as a computer system) through the network device 130 so as to transmit the computing result of the molecular dynamics simulation to the post-computing system 20 for post-computing, such as visual computing, and thus the computing result of the molecular dynamics simulation is converted into 3D images. In some embodiments, the post-computing system 20 may be configured to be a computing system for implementing augmented reality (AR), virtual reality (VR), mixed reality (MR) or extended reality (XR) technology, converting the computing result of the molecular dynamics simulation into AR, VR, MR or XR images, as well as interacting with the user through an device 30 of the user, such as a head-mounted device, a wearable device (such as smart glasses), a tactile feedback device (such as tactile gloves, and tactile clothes) or a combination thereof. The AR overlays the information generated by a computer on an object seen by a user through a smart device (such as a smart phone, a tablet computer or smart glasses) so as to enhance the visual field of the user in the real world. The VR completely replaces the visual field of a user with the information generated by a computer through smart glasses or head-mounted device, so that the user immerses in a virtual environment generated by the computer. For example, the VR enables the user to enter a virtual molecular world, and thus the user can operate and observe molecular interaction in real time, for example, binding or reaction pathway of the target molecule (such as the receptor, like protein) and the test molecule (such as a ligand, like the drug-like molecule). The AR can superpose the molecular structure onto the environment to help the user to visualize complex molecular conformation and experimental settings. The MR combines these methods to allow collaborative exploration of molecular simulation, and enable the user to interact with a dynamic molecular model, thus improving the effect of research and education. These techniques not only improve the spatial understanding of molecular behaviors, but also expedite drug discovery by intuitive manipulation on nanoscale systems, especially the dissociation between the target molecule and the test molecule.

In some embodiments of the method 1 based on FIG. 1 and FIG. 3, when the molecular dynamics simulation process starts, the guiding vector is configured in response to an input indication from a hand interaction action of a user through an application program. In some examples, the application program is an application program that implements one of the augmented reality (AR), virtual reality (VR), mixed reality (MR), or extended reality (XR) technologies. For example, in an embodiment about FIG. 4, the molecular dynamics simulation process and the corresponding stochastic perturbation process are configured to be performed by the computing system 10, and the application program is configured to be executed by the post-computing system 20 to interact with the user. The user configures the guiding vector through the device 30 such as tactile glove using the input indication corresponding to the hand interaction action. For example, the user utilizes images of a virtual molecular model and a reference coordinate frame, points out the direction of the guiding vector in space and sets the magnitude thereof. The application program is used for converting the input indication into the corresponding guiding vector and then transmitting the guiding vector to the computing system 10 so as to set the operation of the molecular dynamics simulation process and related stochastic perturbation process.

According to some embodiments of the present disclosure, a method for applying the adaptively stochastic perturbations in molecular dynamics simulation (ASPMDS) to the atoms of the test molecule is provided. The method for applying the ASPMDS to the atoms of the test molecule (hereinafter referred to as ASPMDS method or ASPMDS) can be applied to the embodiments of stochastic perturbation process in FIG. 1 or FIG. 3, so as to determine the perturbation set (as shown in operation S220) or to obtain the perturbed states of the target molecule and the test molecule (as shown in operation S230). These perturbations are used for controlling the acceleration, velocity or displacement of the physical quantity in motion associated with each atom of the test molecule, thereby facilitating the dissociation of the test molecule from binding pockets. The method uses the following technical features:
1. Perturbation magnitude and perturbation time interval: a stochastic perturbation with constant magnitude is performed at a fixed time interval to ensure systematic and reproducible effects, and facilitate rapid dissociation, as well as preventing non-productive sampling.
2. Perturbation direction: the direction of stochastic perturbation is strategically distributed:
   (1) the stochastic perturbation is adaptively aligned along a pathway from the interior of the pocket of the target molecule to an exit, and is guided by one or more vectors (or referred to as guiding vectors), wherein the guiding vectors may be one of the following:
      A. a guiding vector defined (or configured) by the user;
      B. a vector connecting the center of geometry (CoG) of the target molecule and the center of geometry of the test molecule is treated as the guiding vector; or
      C. a vector connecting the center of mass (CoM) of the target molecule and the center of mass of the test molecule is treated as the guiding vector.
   (2) The stochastic perturbation is distributed within a specified angle relative to the guiding vector, so that the perturbation (or referred to as perturbation vector) fits the spatial geometry of the pocket of the target molecule.
3. Stop conditions: when the distance from the gravity center or the center of mass of the test molecule to the target molecule exceeds a threshold Dₛₜₒₚ, the molecular dynamics simulation will stop. Dₛₜₒₚ is defined as the sum of the initial radius R₁ of the target molecule, the initial radius R₂ of the test molecule and a predefined buffer distance D_{buffer}.

Therefore, with the above improvements, the method can be applied to the computer-implemented molecular dynamics simulation method, thus facilitating screening of various test molecules with respect to the same target molecule. The method may also be used for evaluating the influence of mutation on a protein-protein interface or evaluating membrane-embedded proteins.

An application embodiment 1 and an application embodiment 2 for implementing ASPMDS are described below.

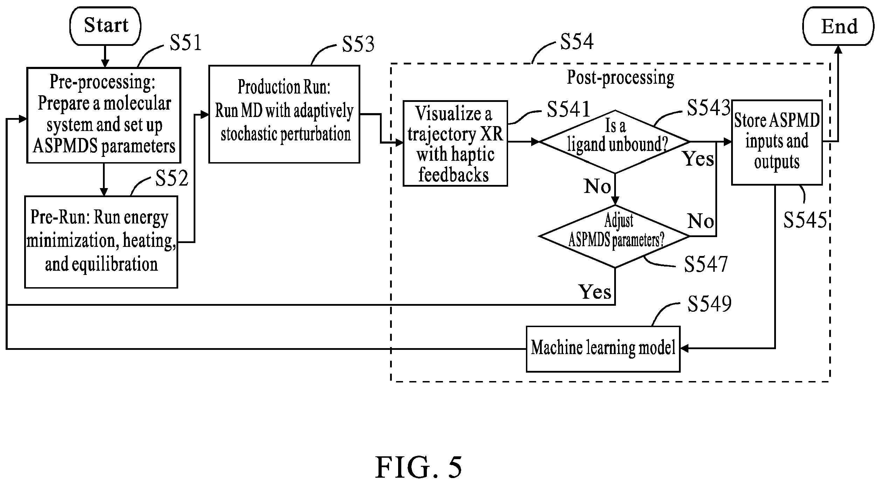

In application embodiment 1, the molecular dynamics simulation (MD) is accompanied with the ASPMDS. In other words, in application embodiment 1, the ASPMDS is directly used when the molecular dynamics simulation (MD) is performed formally from the beginning. As shown in a flowchart in FIG. 5, the workflow in application embodiment 1 includes operations S51 to S54.

Operation S51 includes pre-processing. In this initial phase, molecular systems are combined and prepared for simulation. This includes a plurality of tasks, for example, selecting and clearing the structures of the target molecule and the test molecule, applying force fields, and setting initial parameters (e.g. temperature, pressure, and pH) that are stored as input files with respect to the molecular dynamics simulation. In operation S51, the guiding vector and random parameters with respect to the ASPMDS are set. Operation S52 follows operation S51.

Operation S52 includes pre-run. In operation S52, the energy minimization is run to eliminate any adverse contact, heating is run to make the system reach a target temperature, and equilibration is run to stabilize the system prior to main simulation. Operation S53 follows operation S52.

Operation S53 includes production-run, i.e. running MD with adaptively stochastic perturbation. Operation S54 follows operation S53.

Operation S54 includes post-processing. For example, operation S54 can include the following operations S541 to S549.

Operation S541 includes: visualizing a trajectory XR with haptic feedback. After production-run of operation S53, the generated trajectory can be visualized in an extended reality (XR) environment. The XR definition herein includes virtual reality VR/augmented reality AR/mixed reality MR. This immersive post-processing step provides three-dimensional interactive images of molecular simulation, possibly with tactile feedback and/or hand tracking, for analysis and interpretation.

Operation S543 includes: determining whether the test molecule is not bound. If the result in operation S543 is yes (not bound), operation S545 is performed. If the result in operation S543 is no (bound), operation S547 is performed.

Operation S545 includes: storing ASPMD input and output for further analysis by other tools.

Operation S547 includes: determining whether to adjust ASPMDS parameters. If so, the ASPMDS parameters can be adjusted to improve simulation, followed by returning operation S51 for simulation again.

In operation S549, optionally, the simulation results may be stored (such as operation S545) for use by a machine learning model. The machine learning model can be used for optimizing ASPMDS adjustment to improve the efficiency of the stored ASPMDS input and output. For example, the machine learning can provide input suggestions for ASPMDS parameters and estimate the residence time better.

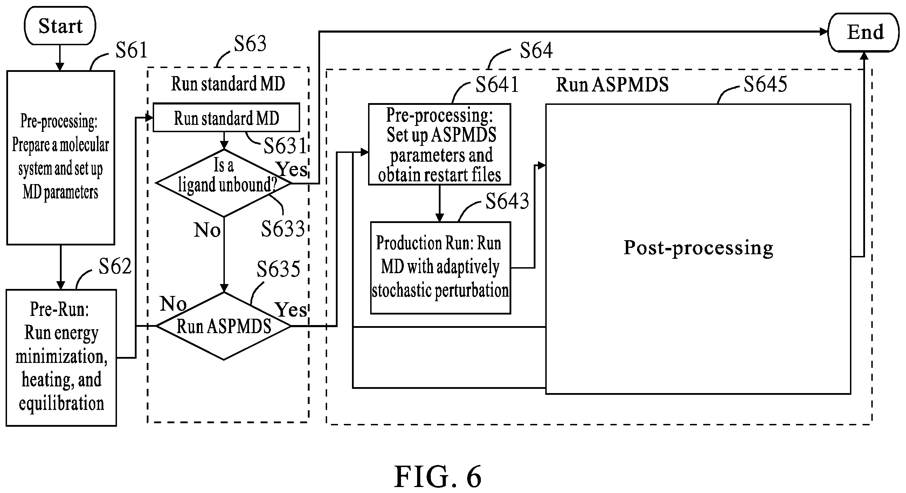

In application embodiment 2, a hybrid approach is adopted. The running of standard MD requires a large amount of simulation time to complete the entire dissociation process. However, before running the ASPMDS, the standard MD can be run to obtain various initial settings as starting points. This hybrid approach represents a molecular dynamics simulation (MD) workflow integrating the adaptively stochastic perturbations in molecular dynamics simulation (ASPMDS). The hybrid approach can improve the accuracy of the ASPMDS and the computing efficiency of standard MD. The hybrid approach shown in FIG. 6 includes the following operations.

Operation S61 includes pre-processing. A molecular system is prepared and MD parameters are set. Operation S62 follows operation S61.

Operation S62 includes pre-run. The energy minimization, heating and equilibration are run to prepare for system simulation. Operation S63 follows operation S62.

Operation S63 includes running the standard MD, including the following operation. In operation S631, standard molecular dynamics (MD) simulation is performed. In the simulation process, the system checks whether the test molecule is not bound, as shown in operation S633. If the test molecule is not bound, the simulation is stopped; otherwise, operation S635 is performed. In operation S635, the user can determine whether to proceed the standard MD or run the ASPMDS. If the user determines to perform the standard MD, the flow returns operation S631; and if the user determines to run the ASPMDS, operation S64 will be performed.

Operation S64 includes: running the ASPMDS, including the following operations.

Operation S641 includes: pre-processing, including setting ASPMDS parameters and obtaining a restart file. Then, operation S643 is performed.

Operation S643 includes production-run, i.e., running MD with adaptively stochastic perturbation.

Operation S645 includes post-processing. For example, operation S645 is the same as operation S54 in application embodiment 2, and operation S645 may include operation S541 to S549 in FIG. 5.

The following examples illustrates the initialization of a perturbation protocol during pre-processing of the ASPMDS.

The perturbation protocol includes 5 characteristic parameters: (1) perturbation physical type, (2) perturbation magnitude, (3) perturbation time interval, (4) perturbation directions, and (5) stop condition.

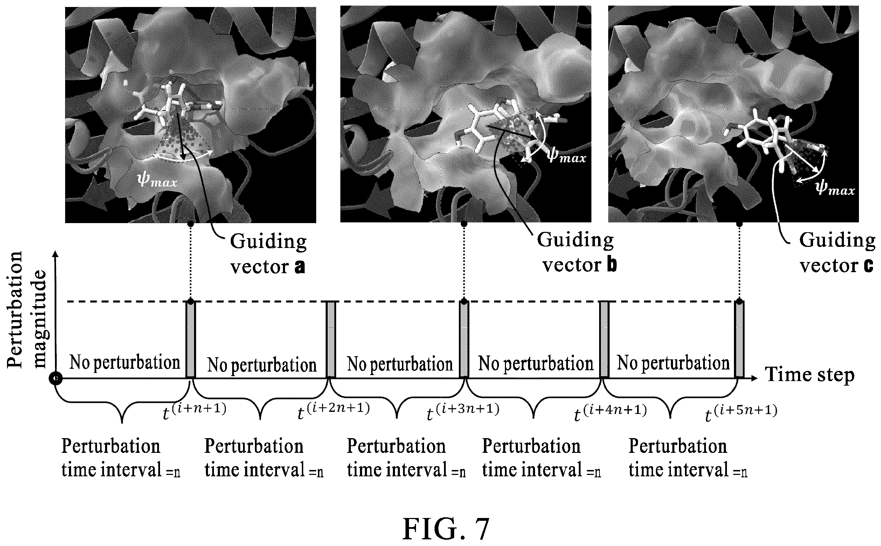

FIG. 7 is a schematic diagram of characteristic parameters of a perturbation protocol. The following paragraphs describe each characteristic parameter.

(1) Perturbation physical type: the perturbation physical type may be acceleration, velocity or displacement.

Adaptively stochastic perturbation *pᵣⁱ* may be one of adaptively stochastic acceleration *aᵣⁱ*, adaptively stochastic velocity *vᵣⁱ* or adaptively stochastic displacement *dᵣⁱ*. The adaptively stochastic perturbation *pᵣⁱ* is applied to each atom of the test molecule or the target molecule to expedite the molecular dynamics simulation process.

In one example, in a case of the adaptively stochastic acceleration, the adaptively stochastic perturbation imparts adaptively stochastic acceleration, therefore, *pᵣⁱ* = *aᵣ*^{(*i*)}. Referring to FIG. 8, it is a schematic diagram of an embodiment of an atom being subject to acceleration perturbation. The left side in FIG. 8 shows molecular dynamics simulation without perturbation between time steps, and the right side in FIG. 8 is molecular dynamics simulation with adaptively stochastic acceleration perturbation between time steps. The schematic diagrams in FIG. 9 and FIG. 10 are similar to the schematic diagram in FIG. 8. As shown in FIG. 8, the adaptively stochastic acceleration *aᵣ*^{(*i*)} is provided to one atom of the test molecule, thus expediting the simulation process of the time step i. a*ᵣ*^{(*i*)} can be updated along a dissociation pathway of the drug-like molecule according to the adaptive guiding vector.

In FIG. 8, the acceleration of the atom after perturbation is a^{(*i*)} = *aᵣ*^{(*i*)} + aᵤ^{(*i*)}. The adaptively stochastic acceleration *aᵣ*^{(*i*)} is along a pathway (dissociation pathway) leading to an exit of the pocket of the target molecule (as illustrated by a pattern right above in FIG. 8). a*ᵣ*^{(*i*)} = *au̅* is a random acceleration vector. a*ᵤ*^{(*i*)} is an acceleration resulting from a force derived from a potential energy function U, *aᵤ*^{(*i*)} = *∇U*/*m,* where m is the mass of the atom.

In another example, in a case of adaptively stochastic velocity, the adaptively stochastic perturbation imparts the adaptively stochastic velocity, therefore, *pᵣⁱ* = *vᵣ*^{(*i*)}. Referring FIG. 9, it is a schematic diagram of an embodiment of atoms being subject to velocity perturbation. Similar to FIG. 8, as shown in FIG. 9, the adaptively stochastic velocity *vᵣ*^{(*i*)} is provided to one atom of the test molecule to expedite the simulation process of time step i. *vᵣ*^{(*i*)} can be updated along a dissociation pathway of the drug-like molecule according to the adaptive guiding vector.

In FIG. 9, the velocity of the atoms after perturbation is *v*^{(*i*)} = *vᵣ*^{(*i*)} + *vᵤ*^{(*i*)}. Where *vᵣ*^{(*i*)} is a random velocity within a cone angle. *vᵣ*^{(*i*)} = *vu̅*is a random velocity vector. *vᵤ*^{(*i*)} is an unperturbed vector.

In another example, in a case of adaptively stochastic displacement, the adaptively stochastic perturbation imparts the adaptively stochastic displacement, therefore, *pᵣⁱ* = *dᵣ*^{(*i*)}. As shown in FIG. 8, the adaptively stochastic displacement *dᵣ*^{(*i*)} is provided to one atom of the test molecule to expedite the simulation process. *dᵣ*^{(*i*)} is a random displacement within a cone angle.

In the above examples, the stochastic perturbation *pᵣⁱ* is adaptively adjusted to direct the test molecule away from the binding pocket or binding site, thus facilitating dissociation along a natural pathway of the pocket.

(2) Perturbation magnitude: it remains unchanged throughout the molecular dynamics simulation process.

(3) Perturbation time interval: it remains unchanged throughout the molecular dynamics simulation process.

(4) Perturbation direction: it is determined based on a stochastic unit vector, where the stochastic unit vector is determined within the range of an angle (denoted as ***ψₘₐₓ*** or Psi_max) along the axis that is the adaptive guiding vector.

(4.1) Initialization of adaptive guiding vectors and an updating mechanism. The "guiding vector" refers to a vector of the pathway from the binding pocket to the unbound exit of the test molecule. The guiding vectors can be adaptively updated according to the situation in the simulation process, so they are referred to as "adaptive guiding vectors". Two embodiments for configuring the adaptive guiding vectors are presented. In addition, the adaptive guiding vectors may also be configured by modifying based on either embodiment or configured in other ways.

(4.1.1) In one embodiment of configuring the adaptive guiding vector, the adaptive guiding vector is configured by the user.

For example, as shown in FIG. 7, according to the conformation of the test molecule and the target molecule, in an XR environment, the user can provide the adaptive guiding vector, such as three adaptive guiding vectors a, b, and c in sequence. In some examples, the user can directly configure the guiding vectors with hands in the XR environment. For example, the user makes a predetermined related gesture, such as clicking with thumb and another finger, and an artificially intelligent camera in the XR environment can recognize the gesture and trigger a hand tracking camera to record 3D coordinates of the clicked position. Finally, an XR application shows a starting point at the clicked position. Then, the finger is dragged to a target point and clicks again. The XR application will draw a 3D arrow from the starting point to the target point. However, the implementation of the method is not limited by these examples.

In addition, the updating mechanism of the adaptive guiding vector is as follows. At the beginning, the adaptive guiding vector is set as a. When the center of the test molecule enters a specified region around a starting point of the guiding vector b, the guiding vector a is updated to the guiding vector b. Then, if the test molecule moves into a specified region around a starting point of the guiding vector c, the guiding vector b is updated to the guiding vector c. In the embodiment of the present disclosure, the "center" of the molecule is understood to be one of the center of mass (CoM) or the center of geometry (CoG). For clearness and conciseness, the term "center" used in the whole description covers the meaning of the center of mass or the center of geometry.

(4.1.2) In another embodiment for configuring the adaptive guiding vector, the adaptive guiding vector is configured in a preset mode.

The initial adaptive guiding vector is configured (or defined) as a vector from the center of the target molecule to the test molecule in initial configuration. The adaptive guiding unit vector *u*⁰*ᵥ* is used for facilitating dissociation along the natural pathway of the pocket or binding site. The adaptive guiding unit vector *u*⁰*ᵥ* is dynamically updated according to the following conditions:

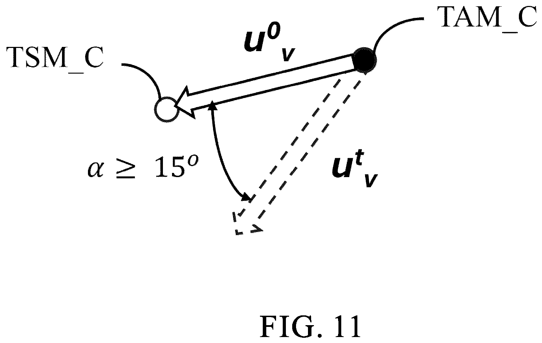

If the inner products of *u*⁰*ᵥ* and *u^{t}ᵥ* meet the following inequality: ${{u}^{0}}_{v} ⋅ {{u}^{t}}_{v} \geq cos \left(α\right)$
where *α* is 15°, 30° or a user-defined angle, then, ${{u}^{0}}_{v} = {{u}^{t}}_{v} .$
*u^{t}ᵥ* represents a unit vector of time t from the target molecule to the drug-like molecule. FIG. 11 is a schematic diagram of an embodiment of adaptively updating a guiding vector, where TAM_C represents the center of the target molecule, and TSM_C represents the center of the test molecule.

(4.2) The Psi_max angle is initialized for generating a stochastic unit vector according to the adaptive guiding vector. A group of stochastic unit vectors are generated using the Psi_max angle, and these vectors are within the range of the Psi_max angle around the adaptive guiding vector *u*⁰*ᵥ*.

(4.2.1) In one example, the user can set the Psi_max angle: the Psi_max angle may be provided by user interaction settings in the XR environment. For example, three different Psi_max angles corresponding to the sequential adaptive guiding vectors a, b, and c are represented with arc arrows in FIG. 7. Different settings of Psi_max may produce different stochastic unit vector distributions. The stochastic unit vectors may be produced in a spherical coordinate system.

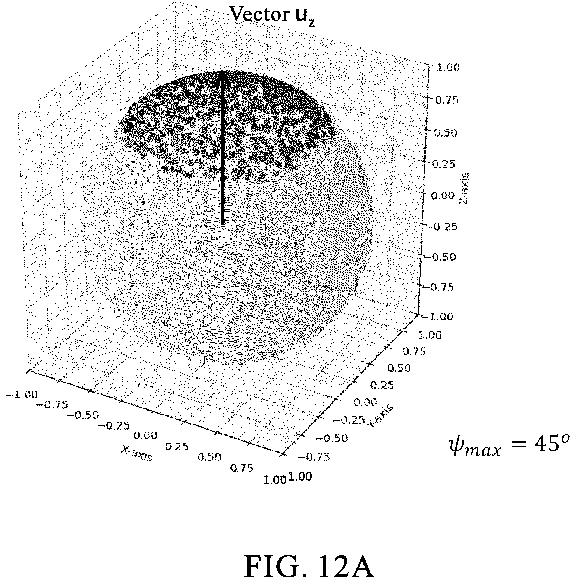

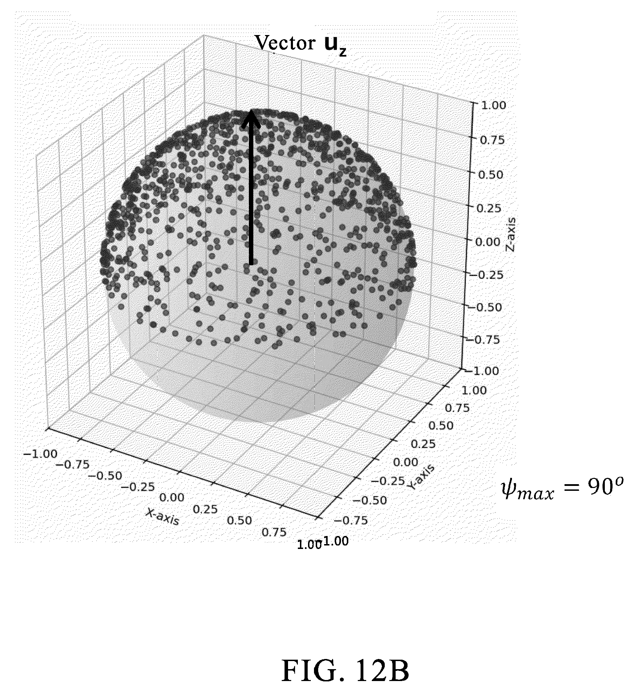

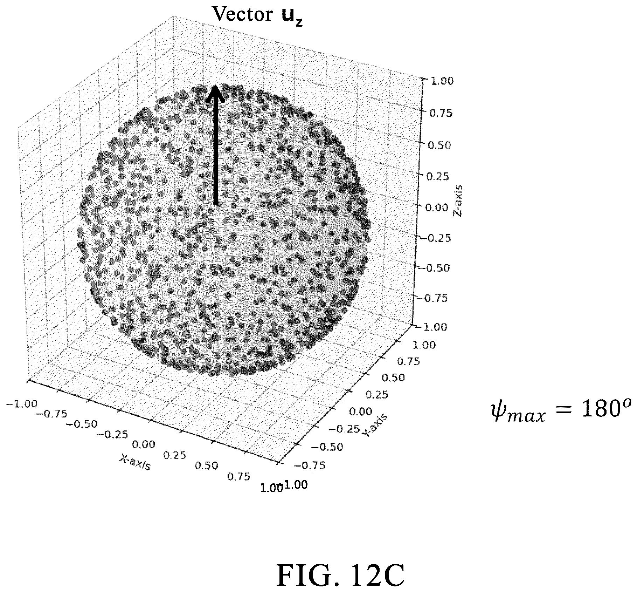

For example, *u*⁰*ᵥ* = *u_{z}* = (0,0,1). FIG. 12A, FIG. 12B, and FIG. 12C show different distributions of the stochastic unit vectors relative to Psi_max angles at 45°, 90°, and 180°. In FIG. 12A to FIG. 12C, bold arrows represent the vector *u_{z}*, while points distributed around the vector *u_{z}* and a starting point of the vector *u_{z}* may produce a plurality of different numbers (e.g., 1000 or suitable numbers) of stochastic unit vectors.

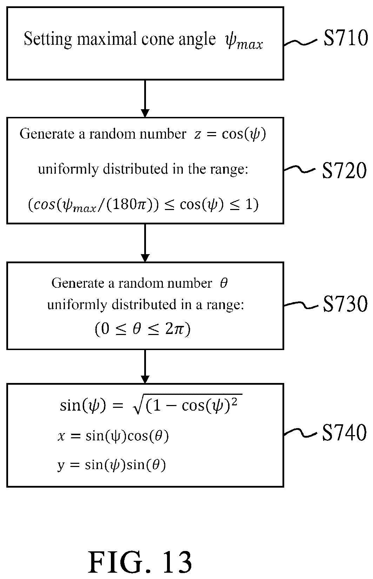

(4.2.2) In another example, a preset Psi_max angle is used: throughout the simulation process, the Psi_max angle is kept unchanged, for example, about 90°. After the Psi_max angle is initialized, a stochastic unit vector (x, y, z) can be generated in the spherical coordinate system, as shown in FIG. 13. The random numbers between a lower limit = cos(Psi_max/(180*Pi)) and an upper limit =1 are uniformly distributed and are assigned to cos(Psi).

Operation S710 includes: setting the maximum cone angle *ψₘₐₓ* . That the unit of the cone angle is degree is taken as an example.

Operation S720 includes: generating a random number *z* = cos(*ψ*) that is uniformly distributed in a range (*cos*(*ψₘₐₓ*/(180*π*)) ≤ cos(*ψ*) ≤ 1).

Operation S730 includes: generating a random number *θ* that is uniformly distributed in a range (0 ≤ *θ* ≤ *2π*).

Operation S740 includes: calculating to convert spherical coordinates into rectangular coordinates: $sin \left(ψ\right) = \sqrt{\left(1-cos {\left(ψ\right)}^{2}\right.}$, *x* = sin(*ψ*)cos(*θ*), y = sin(*ψ*)sin(*θ*)

(5) A stop condition is set, and the dissociation condition can be determined by the following formula: if Dₜ >= Dₛₜₒₚ, then separation is determined and simulation is stopped. Dₜ is equal to the distance from the center of the test molecule and the target molecule at time t, Dₛₜₒₚ = R₁ + R₂ + D_{buffer} is used for setting the maximum distance that the test molecule is determined to be completely dissociated from the target molecule. R₁ is equal to the maximum distance from the center of the target molecule to the atomic center thereof in an initial configuration, R₂ is equal to the maximum distance from the center of the test molecule to the atomic center thereof in an initial configuration, D_{buffer} is equal to the buffer distance, and therefore, a situation that there is still an interaction between the target molecule and the test molecule due to conformational changes or other factors can be avoided. The buffer distance may be set to 5-10Å. However, in some cases, the buffer distance can be adjusted. For example, due to the differences in drug-target systems, the buffer distance can be adjusted when performing relevant molecular dynamics simulations.

The following exemplifies a formal operation workflow in ASPMDS.

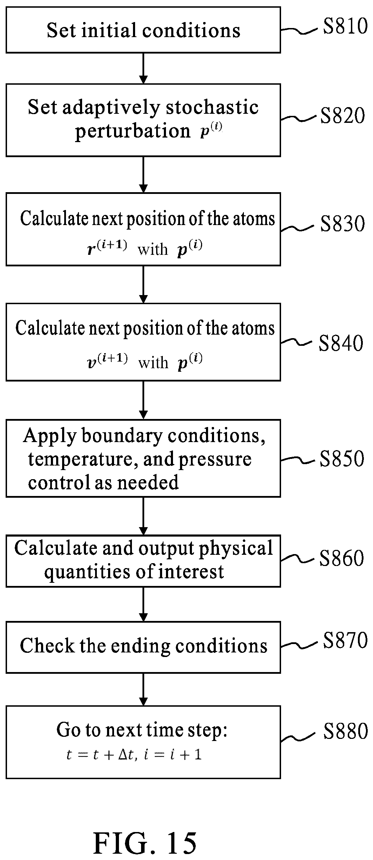

After the ASPMSD protocol is established during ASPMDS pre-processing, formal operation of ASPMDS can be performed. As shown in FIG. 15, an embodiment of the ASPMSD workflow includes the following operations.

Operation S810 includes: setting initial conditions. For example, an initial position *r*^{(*i*=0)}, an initial velocity *v*^{(*i*=0)}, and an initial acceleration *a*^{(*i*=0)} of the atom are set; and at t = 0.0, Δt = 0.002 picoseconds (ps).

Operation S820 includes: setting adaptively stochastic perturbation ***p***^{(*i*)}. For example, an interval of the time steps between consecutive perturbations is set to 500 or 2000; the magnitude of constant perturbation is set to 10000; the Psi_max angle is set to 90 ° or a user setting value; and the adaptive guiding vector is set.

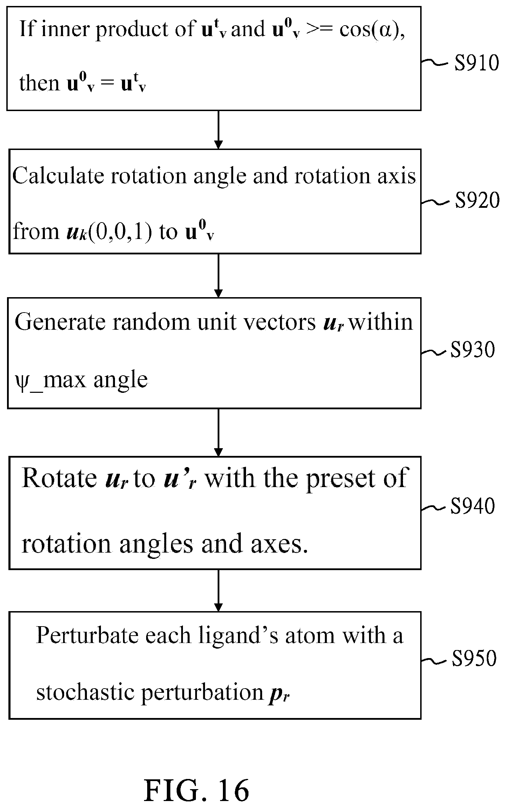

Operation S830 includes: calculating a next position ***r***^{(***i*+1**)} of an atom through the adaptively stochastic perturbation ***p***^{(*i*)}. For example, as shown in FIG. 16, adaptive perturbation is generated. For example, depending on related correlation parameters including the adaptively stochastic perturbation ***p***^{(***i***)}, the next position ***r***^{(***i*+1**)} is generated using a function f (*), and is represented as follows with symbols: ${r}^{\left(i+1\right)} = f \left({r}^{\left(i\right)},{v}^{\left(i\right)},{a}^{\left(i\right)},{p}^{\left(i\right)},Δt\right) ,$ where f (*) depends on integrators used.

Operation S840 includes: calculating the next velocity ***v***^{(***i*+1**)} of the atom using ***p***^{(***i***)}. For example, depending on related parameters including the adaptively stochastic perturbation ***p***^{(***i***)}, the next velocity ***v***^{(***i*+1**)} is generated using the function g(*), and is represented as follows with symbols: ${v}^{\left(i+1\right)} = g \left({v}^{\left(i\right)}, {a}^{\left(i\right)}, {p}^{\left(i\right)}\right) ,$ where g (*) depends on the integrators used.

Operation S850 includes: using boundary conditions, temperature and pressure controls as required.

Operation S860 includes: calculating and outputting physical quantities of interest.

Operation S870 includes: checking stop conditions. For example, if the simulation stop condition (such as Dₜ >= Dₛₜₒₚ) is satisfied, the simulation will be stopped. For another example, when the number of the time steps of the simulation reaches the maximum number of allowable steps, the simulation will be stopped.

Operation S880 includes: entering the next time step: t=t+Δt, i=i+1.

Referring to FIG. 16, it is a flowchart of an embodiment of producing adaptive perturbation, and it is applicable to the workflow of the above formal operation as shown in FIG. 15. As shown in FIG. 16, the production of adaptive perturbation includes the following operations.

Operation S910 includes: if the inner product of **u^{t}v** and **u⁰ᵥ** is >= cos(α), **u⁰ᵥ** = **u^{t}ᵥ**.

Operation S920 includes: calculating an angle of rotation and an axis of rotation from ***uₖ***(0,0,1) to ***u⁰ᵥ***.

Operation S930 includes: generating a stochastic unit vector ***uᵣ*** within the ψ_max angle.

Operation S940 includes: rotating ***uᵣ*** to ***u'ᵣ**,* according to preset angle of rotation and axis of rotation.

Operation S950 includes: perturbing atoms of each ligand using stochastic perturbation ***pᵣ***.

The efficacy according to embodiments of the present disclosure is illustrated by the following comparative embodiments.

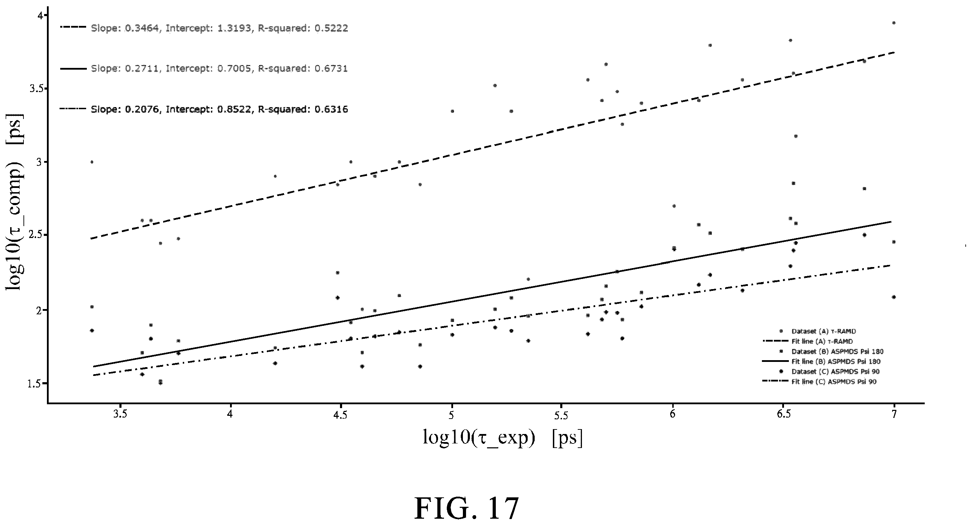

In the following comparative embodiments, preferred embodiments of ASPMDS relate to the implementation of encoding in AMBER 22 software kits for estimating the residence time of the drug-like molecules.
1. Pre-processing
   Preparation of an N-HSP90 complex by established models and force field tools:
   (1) Select 31 compounds and crystal structures thereof from a protein database.
   (2) Perform structure repair through MODELLER software.
   (3) Perform partial charge computing of restrained electrostatic potential (RESP) by Gaussian09 and antechamber.
   (4) Perform system dissolution by an AMBER LEaP module using TIP3P water and counter ions.
   (5) Apply a stochastic perturbation with constant magnitude of 10,000 kcal/mol-Å to every 1,000 time steps. The perturbation direction is distributed within the Psi_max angle of 90° or 180°. The drug-like molecule dissociation pathway is limited in an outward direction using the Psi_max angle in simulation.
2. Pre-run: energy minimization and balance are realized by a Langevin thermostat. Energy minimization is performed by 1 kcal/mol harmonic constraint on solute atoms, and then balance at 300K is achieved by the Langevin thermostat with collision frequency of 2 ps⁻¹.
3. Production run: ASPMDS is run by Linux versions of GPU 4090 and Amber 22 PMEMD.
4. Post-processing: (1) The track is verified in XR after the simulation is finished so as to verify the occurrence of dissociation. (2) The dissociation time is determined by multiplying the total number of time steps by a time step interval (Δt = 0.002 ps). The dissociation time is related to the residence time; therefore, a linear regression model is constructed to establish a relationship between the dissociation time (expressed as τ_comp) in a computer experiment and the residence time (expressed as τ_exp) in a physical experiment. The estimation accuracy of τ_comp with respect to the residence time τ_exp can be represented by a slope and R² of the linear regression model. FIG. 17 shows regression models of τ-RAMD, ASPMDS-90 and ASPMDS-180.

Table 1 shows the result comparison among ASPMDS with Psi_max = 180 and ASPMDS with Psi_max = 90 in the embodiment and τ-RAMD in the paper of Daria B. The paper of Kokh (2018). Since the value of τ_comp is only related to the complexes (the target molecule and the drug-like molecule) and perturbation protocols, and is not related to the encoding on computing hardware and MD software kit software (such as AMBER or NAMD). According to the results in Table 1 and Table 2, it is known that the average τ_comp of ASPMDS-90 is only 10.13% of τ-RAMD required for predicting residence time of τ-RAMD, and the error of prediction accuracy and τ-RAMD is R² = 0.63 (ASPMDS-180 is R² = 0.67), and = 0.52), and R² is the greater the better.

Table 1 shows the dissociation time comparison of τ-RAMD, ASPMDS-180, and ASPMDS 90. The data set is 35 complexes selected from N-HSP90.

**Table 1**

| Perturbation solutions | | Ratio of average τ _comp | R² in linear regression |
|---|---|---|---|
| τ-RAMD | | 100.00% | 0.52 |
| | •Perturbation Type: Acceleration | | |
| | •Perturbation magnitude: 14.7 | | |
| | •Perturbation time interval: 0 | | |
| | •Psi_max = None | | |
| ASPMDS-180 | | 14.12% | 0.67 |
| | •Perturbation Type: Acceleration | | |
| | •Perturbation magnitude: 10,000 | | |
| | •Perturbation time interval: 1,000 | | |
| | • Psi_max = 180 | | |
| ASPMDS-90 | | 10.13% | 0.63 |
| | •Perturbation Type: Acceleration | | |
| | •Perturbation magnitude: 10,000 | | |
| | •Perturbation time interval: 1,000 | | |
| | • Psi_max = 90 | | |

Table 2: Dissociation times of τ-RAMD, ASPMDS-180, and ASPMDS 90.

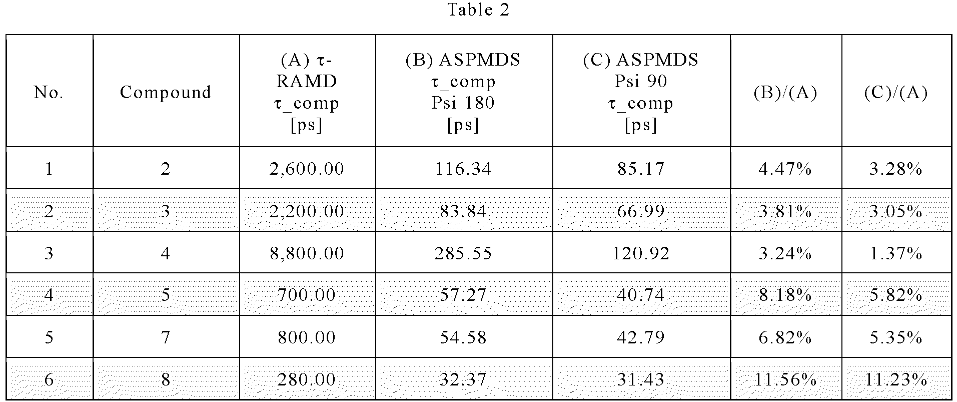

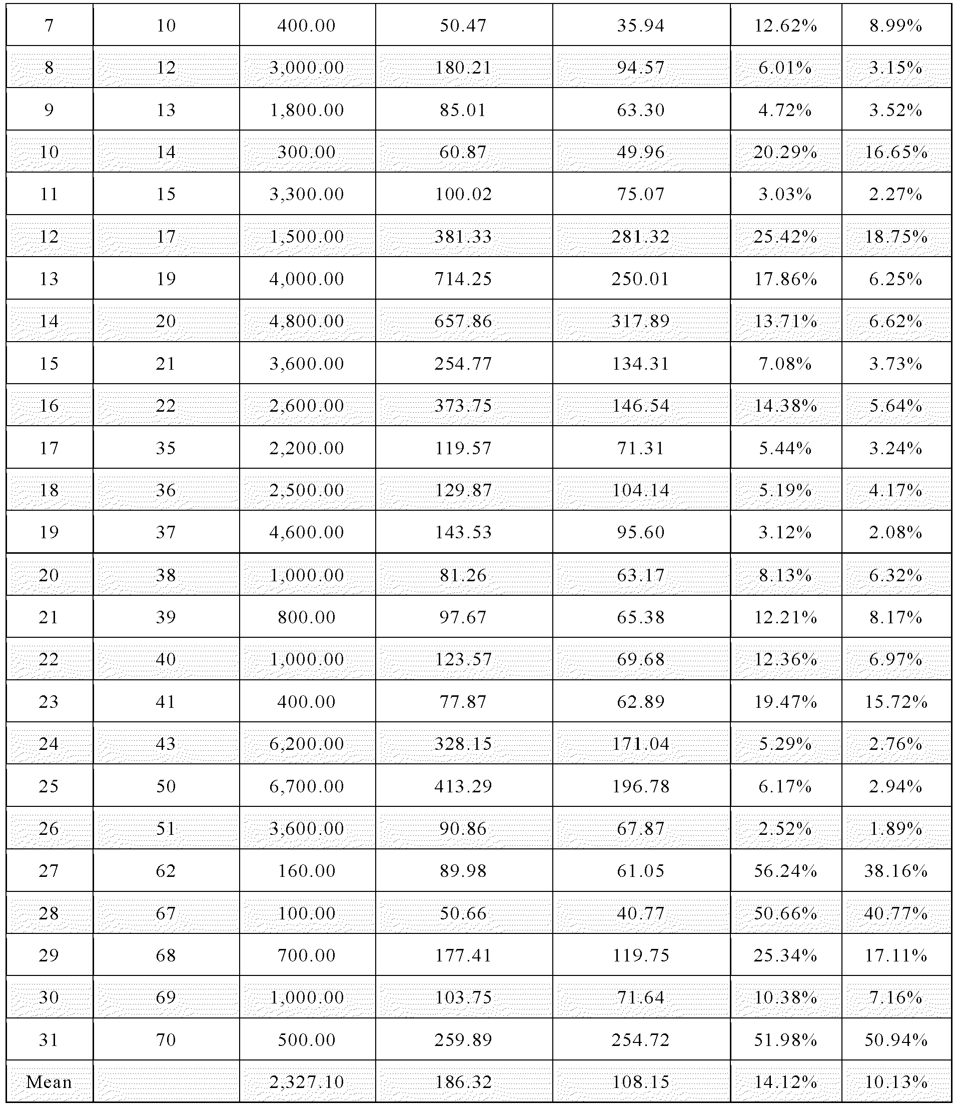

In addition, disclosed is a method according to the embodiments of the present disclosure. The method can be applied to the embodiments of stochastic perturbation process in FIG. 1 or FIG. 3 so as to determine the perturbation set (such as operation S220) or obtain the perturbed states of the target molecule and the test molecule (such as operation S230). The method includes the following step (a) to step (e):
(a) Acquire a pre-perturbation state: acquiring initial states of the target molecule and drug-like molecule before any perturbation.
(b) Determine perturbation conditions: establishing perturbed conditions of the drug-like molecule.
(c) Perturb and obtain the perturbed states: applying perturbation to the molecules according to the determined perturbation condition and acquiring the perturbed states of the molecules.
(d) Determine a dissociation condition: evaluating whether the molecules satisfy the predefined dissociation condition relative to the target molecule.
(e) Repeat the process: if the dissociation condition is not satisfied, waiting for a preset perturbation time interval and returning to step (a) to repeat the above processes.

In an embodiment of the method, the determining perturbation conditions includes: determining the perturbed conditions of the molecules. The perturbation conditions include determining the direction of the guiding vector, which may be any one of the following:
1. User-defined: the user specifies the direction of the guiding vector.
2. Vector based on the center of geometry: the direction of the guiding vector is determined based on the vector from the target molecule to the center of geometry of the drug-like molecule.
3. Vector based on the center of mass: the direction of the guiding vector is determined according to the center of mass vector from the target molecule to the drug-like molecule.

In an embodiment of the method, when the perturbation direction is based on the guiding vector, the method may include:
Generate stochastic unit vectors: randomly determining a group of vectors having a predetermined angle with respect to a reference direction (0,0,1).
Rotate the stochastic unit vectors: rotating a random vector set according to the reference direction of the guiding vector.
Limit angle: ensuring that an angle between each random vector and the reference direction is less than the preset angle Psi_max.
Number of the stochastic unit vectors: the number of the stochastic unit vectors corresponds to the number of atoms in the molecules.

In addition, in an embodiment of the method, the method may include:
Set perturbation attributes: defining the property of perturbation, which may be velocity, acceleration or displacement.
Set perturbation magnitude: specifying the constant amplitude of perturbation to be applied.
Set perturbation time interval: determining the frequency of applying the perturbation in the constant time interval.

In an embodiment of the method, the determining dissociation conditions (stop conditions) include: evaluating whether the drug-like molecule satisfy the predefined dissociation conditions relative to the target molecule or not. The example of this situation is as follows: the stop condition based on distance is: Dₜ > Dₛₜₒₚ and Dₛₜₒₚ = R₁ + R₂ + D_{buffer}, where Dₜ represents the distance from the target molecule to the drug-like molecules at the time t, R₁ and R₂ are the initial radiuses of the target molecule and the drug molecule respectively, and D_{buffer} is set to 5-10Å.

In an embodiment of the method, if the above conditions are satisfied, simulation is stopped, and dissociation time = time step number multiplied by delta Δt = 0.002 ps is outputted.

In some embodiments, a computer program product is proposed, which includes a plurality of instructions, and the plurality of instructions are used for causing the computer to perform at least one of a plurality of embodiments of the computer-implemented method for facilitating molecular dynamics simulation for drug discovery as described above. In some embodiments, the computer program product may include a storage medium, such as a non-transient storage medium storing instructions (or program codes) that can be read by a computing device, and when executed by at least one computing device, the instructions cause the at least one computing device to implement at least one of a plurality of embodiments of the foregoing method. This method may be one or any combination of all of the above embodiments encompassing the method based on FIG. 1. For example, a program code is one or more programs or program modules, such as those used for implementing operations S10 to S20 based on FIG. 3 (or methods based on at least one embodiment of FIG. 1, FIG. 2, and FIG. 3 or a combination thereof), and may be executed in any appropriate order. When the computing device (such as the preceding network controller 200) executes this code, it may cause the computing device to execute the embodiment of the operating method based on FIG. 1. The embodiments of these storage media include, but are not limited to, optical information storage media, magnetic information storage media or memory, such as memory cards, firmware or ROM or RAM. In some embodiments, the computing device may execute the computing system and may be further implemented by using one or more of the appropriate network or software technologies, such as a network service, a script engine, a web application or a web application programming interface (API) server.

The present disclosure has been disclosed in a plurality of embodiments above, but those skilled in the art should understand that this embodiment is only used for describing the present disclosure and should not be construed as limiting the scope of the present disclosure. It is to be noted that all variations and substitutions equivalent to these embodiments shall fall within the scope of the present disclosure. Therefore, the scope of protection of the present disclosure shall be defined by the scope of the patent application.

## Claims

1. A computer-implemented method for facilitating a molecular dynamics simulation for drug discovery, comprising:
performing a molecular dynamics simulation process with respect to a target molecule and a test molecule until a dissociation condition of the molecular dynamics simulation process is satisfied; and
adaptively performing a stochastic perturbation process during the performing of the molecular dynamics simulation process, wherein the stochastic perturbation process comprises:
obtaining states of the target molecule and the test molecule in the molecular dynamics simulation process before performing the stochastic perturbation process;
determining a perturbation set based on the states of the target molecule and the test molecule; and
performing perturbation on the target molecule and the test molecule according to the perturbation set to obtain perturbed states of the target molecule and the test molecule;
wherein the molecular dynamics simulation process proceeds according to the perturbed states of the target molecule and the test molecule;
wherein in response to that the dissociation condition is not satisfied, the stochastic perturbation process is performed at a perturbation time interval in the performing of the molecular dynamics simulation process, so as to expedite the molecular dynamics simulation process.

2. The computer-implemented method according to claim 1, wherein the perturbation set comprises a group of acceleration stochastic perturbations for each atom of the test molecule.

3. The computer-implemented method according to claim 1, wherein the perturbation set comprises a group of velocity stochastic perturbations for each atom of the test molecule.

4. The computer-implemented method according to claim 1, wherein the perturbation set comprises a group of displacement stochastic perturbations for each atom of the test molecule.

5. The computer-implemented method according to claim 1, wherein the perturbation set comprises a group of stochastic perturbations, and each stochastic perturbation in the group of stochastic perturbations has a same perturbation magnitude.

6. The computer-implemented method according to claim 5, wherein each stochastic perturbation in the group of stochastic perturbations is distributed within a cone angle with respect to the test molecule based on a guiding vector.

7. The computer-implemented method according to claim 1, wherein the perturbation set comprises a group of stochastic perturbations that are distributed within a cone angle of the test molecule and compatible with a pocket of the target molecule.

8. The computer-implemented method according to claim 1, wherein the perturbation set comprises a group of stochastic perturbations that are randomly generated based on a guiding vector and adaptively distributed along a pathway departing from a pocket of the target molecule, the guiding vector is a configurable vector .

9. The computer-implemented method according to claim 8, wherein when the molecular dynamics simulation process starts, the guiding vector is configured in response to an input indication of a user through an application program.

10. The computer-implemented method according to claim 8, wherein the guiding vector is an adaptive guiding vector, and the adaptive guiding vector is updated to a first guiding vector when the test molecule enters a region with respect to a starting point of the first guiding vector.

11. The computer-implemented method according to claim 10, wherein after the adaptive guiding vector is updated to the first guiding vector, the adaptive guiding vector is further updated to a second guiding vector when the test molecule enters a region with respect to a starting point of the second guiding vector.

12. The computer-implemented method according to claim 8, wherein the guiding vector is a vector from a center of geometry of the target molecule to a center of geometry of the test molecule.

13. The computer-implemented method according to claim 8, wherein the guiding vector is a vector from a center of mass of the target molecule to a center of mass of the test molecule.

14. The computer-implemented method according to claim 1, wherein the dissociation condition is whether an inequality Dₜ > Dₛₜₒₚ is satisfied, wherein Dₛₜₒₚ = R₁ + R₂ + D_{buffer}, Dₜ represents a distance from a center point of the target molecule to a center point of the test molecule at a time point in the performing of the molecular dynamics simulation process; R₁ represents a maximum distance from the center point of the target molecule to an outermost atom of the target molecule when the molecular dynamics simulation process starts; R₂ represents a maximum distance from the center point of the test molecule to an outermost atom of the test molecule when the molecular dynamics simulation process starts; and D_{buffer} represents a buffer distance.

## Amended claims

### Amended claims in accordance with Rule 137(2) EPC.

1. A computer-implemented method for facilitating a molecular dynamics simulation for drug discovery, comprising:
performing a molecular dynamics simulation process with respect to a target molecule and a test molecule until a dissociation condition of the molecular dynamics simulation process is satisfied; and
adaptively performing a stochastic perturbation process during the performing of the molecular dynamics simulation process, wherein the stochastic perturbation process comprises:
obtaining states of the target molecule and the test molecule in the molecular dynamics simulation process before performing the stochastic perturbation process;
determining a perturbation set based on the states of the target molecule and the test molecule; and
performing perturbation on the target molecule and the test molecule according to the perturbation set to obtain perturbed states of the target molecule and the test molecule; wherein
the molecular dynamics simulation process proceeds according to the perturbed states of the target molecule and the test molecule;
in response to that the dissociation condition is not satisfied, the stochastic perturbation process is performed at a perturbation time interval in the performing of the molecular dynamics simulation process, so as to expedite the molecular dynamics simulation process; and
the perturbation set comprises a group of stochastic perturbations, and each stochastic perturbation in the group of stochastic perturbations has a same perturbation magnitude.

2. The computer-implemented method according to claim 1, wherein the group of stochastic perturbations is a group of acceleration stochastic perturbations for each atom of the test molecule.

3. The computer-implemented method according to claim 1, wherein the group of stochastic perturbations is a group of velocity stochastic perturbations for each atom of the test molecule.

4. The computer-implemented method according to claim 1, wherein the group of stochastic perturbations is a group of displacement stochastic perturbations for each atom of the test molecule.

5. The computer-implemented method according to claim 1, wherein each stochastic perturbation in the group of stochastic perturbations is distributed within a cone angle with respect to the test molecule based on a guiding vector.

6. The computer-implemented method according to claim 1, wherein the group of stochastic perturbations is distributed within a cone angle of the test molecule and compatible with a pocket of the target molecule.

7. The computer-implemented method according to claim 1, wherein the group of stochastic perturbations is randomly generated based on a guiding vector and adaptively distributed along a pathway departing from a pocket of the target molecule, the guiding vector is a configurable vector.

8. The computer-implemented method according to claim 7, wherein when the molecular dynamics simulation process starts, the guiding vector is configured in response to an input indication of a user through an application program.

9. The computer-implemented method according to claim 7, wherein the guiding vector is an adaptive guiding vector, and the adaptive guiding vector is updated to a first guiding vector when the test molecule enters a region with respect to a starting point of the first guiding vector.

10. The computer-implemented method according to claim 9, wherein after the adaptive guiding vector is updated to the first guiding vector, the adaptive guiding vector is further updated to a second guiding vector when the test molecule enters a region with respect to a starting point of the second guiding vector.

11. The computer-implemented method according to claim 7, wherein the guiding vector is a vector from a center of geometry of the target molecule to a center of geometry of the test molecule.

12. The computer-implemented method according to claim 7, wherein the guiding vector is a vector from a center of mass of the target molecule to a center of mass of the test molecule.

13. The computer-implemented method according to claim 1, wherein the dissociation condition is whether an inequality Dₜ > Dₛₜₒₚ is satisfied, wherein Dₛₜₒₚ = R₁ + R₂ + D_{buffer}, Dₜ represents a distance from a center point of the target molecule to a center point of the test molecule at a time point in the performing of the molecular dynamics simulation process; R₁ represents a maximum distance from the center point of the target molecule to an outermost atom of the target molecule when the molecular dynamics simulation process starts; R₂ represents a maximum distance from the center point of the test molecule to an outermost atom of the test molecule when the molecular dynamics simulation process starts; and D_{buffer} represents a buffer distance.
